Europäisches Patentamt

European Patent Office

Office européen des brevets

(11) Publication number: **0 069 565**
**B1**

(12) **EUROPEAN PATENT SPECIFICATION**

(45) Date of publication of patent specification: **13.03.85**

(51) Int. Cl.⁴: **A 61 B 17/36**

(21) Application number: **82303498.8**

(22) Date of filing: **02.07.82**

(54) **Laser scalpel.**

(30) Priority: **07.07.81 JP 100113/81 U**

(43) Date of publication of application:
**12.01.83 Bulletin 83/02**

(45) Publication of the grant of the patent:
**13.03.85 Bulletin 85/11**

(84) Designated Contracting States:
**DE FR GB SE**

(56) References cited:
**DE-A-2 145 526**
**DE-A-2 530 478**
**DE-B-1 022 354**
**US-A-3 913 582**

(73) Proprietor: **SUMITOMO ELECTRIC INDUSTRIES LIMITED**
**No. 15, Kitahama 5-chome Higashi-ku**
**Osaka-shi Osaka-fu (JP)**

(72) Inventor: **Sunago, Katsuyoshi c/o Osaka Works Sumitomo Electric Ind. Ltd. 1-3, Shimaya 1-chome**
**Konohana-ku Osaka (JP)**
Inventor: **Takenaka, Shinya c/o Osaka Works Sumitomo Electric Ind. Ltd. 1-3, Shimaya 1-chome**
**Konohana-ku Osaka (JP)**

(74) Representative: **George, Sidney Arthur et al**
**GILL JENNINGS & EVERY 53-64 Chancery Lane**
**London WC2A 1HN (GB)**

Courier Press, Leamington Spa, England.

## Description

This invention relates to a laser scalpel in which optical fibres are used as light guides.

One practical use of a laser is as a laser scalpel for use in the medical field, particularly for medical treatments and operations on minute areas requiring high accuracy.

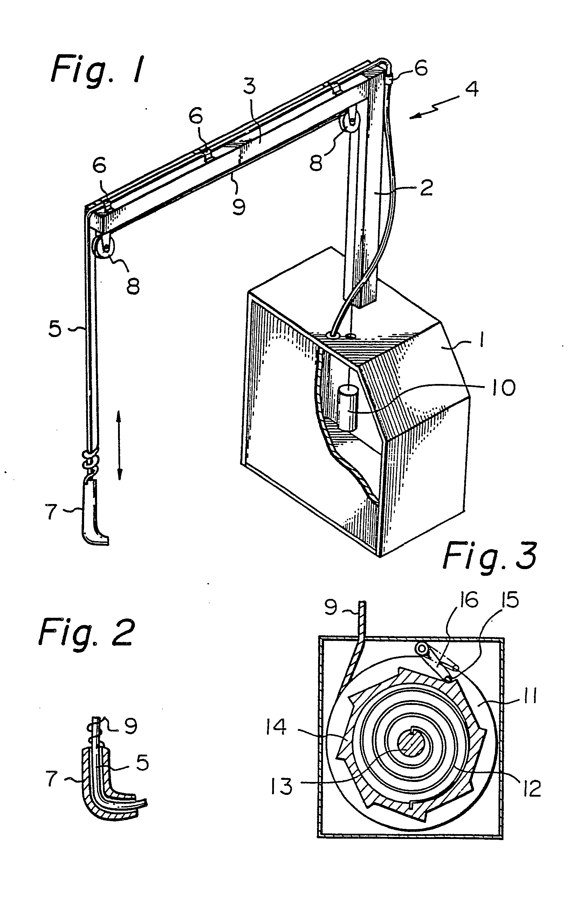

Light and flexible optical fibres are now frequently used in laser scalpels instead of the conventional optical wave guides of the reflecting mirror type. Although Fig. 1 of the accompanying drawing is intended to illustrate a laser scalpel according to the present invention, certain parts of that figure can be used to describe a known form of laser scalpel. Such parts comprise a laser main body 1 in which a laser oscillator, operating as a laser source, and associated equipment are housed, and a support 4 consisting of a post 2 extending vertically from the unit 1 and an arm 3 which extends horizontally and is fixed to the post 2 at one end thereof. An optical fibre 5 is guided along the support 4 and is fixed thereto by means of clips 6. One end of the fibre receives light from the laser and the other end of the fibre is suspended from the free end of the arm 3.

Although the known laser scalpel is used by grasping the free end of the optical fibre 5 or by mounting the free end on an endoscope, it is difficult to direct the laser in directions other than the axial direction of the optical fibres and, therefore, the laser beam cannot be directed accurately, and use of the known laser scalpel is limited as regards the shapes and positions of the portions which can be irradiated. In addition, as the optical fibre 5 is always suspended from the free end of the arm 3, it gets in the way when the laser scalpel is not being used during the operation.

An object of the present invention is to provide a laser scalpel in which these disadvantages of the conventional laser scalpel are alleviated and the optical fibres do not get in the way when the laser scalpel is not being used.

According to the invention, a laser scalpel in which an optical fibre is used as an optical wave guide, and the optical fibre is guided, from a unit housing a laser source to areas to be irradiated, along support means provided on the unit, is characterised in that the optical fibre is provided with a handpiece on the exterior of its free end portion, the handpiece being constructed to facilitate the directing of the free end of the fibre toward the areas to be irradiated; and supporting wire means is provided along the support means so as to be axially movable, one end of the wire means being connected to the handpiece and the other end of the wire means being connected to means for counter-balancing at least the weight of the handpiece.

Embodiments of the invention will now be described, by way of example, with reference to the accompanying drawing, in which:

Fig. 1 is a partly cut-away perspective view showing a preferred embodiment of a laser scalpel according to the present invention,

Fig. 2 is a longitudinal cross section of a handpiece of the unit, and

Fig. 3 is a transverse cross section showing an alternative form of a counter-balance means for the unit.

As described above in relation to a known laser scalpel, an optical fibre 5 is guided along a support 4, which comprises an upright post 2 attached to a unit 1 which houses the laser and associated equipment. A horizontal arm 3 is attached to the post 2. The fibre 5 is attached to the support 4 by clips 6. The free end of the fibre is suspended from the free end of the arm 3.

In an embodiment of the present invention, a handpiece 7 to be grasped by an operator's hand is provided on the free end of the fibre 5. The handpiece 7 is fixed to the outside of the optical fibre 5 by means of adhesive. As shown in Fig. 2, the handpiece 7 has an inside diameter slightly larger than the outside diameter of the optical fibre 5. It can be constructed in various shapes so that the forward end of the fibre may be easily directed to the areas to be irradiated. For example, the fibre may be bent in the shape of the letter L. The use of the handpiece 7 makes it possible to direct the laser beam easily to areas which cannot be irradiated by the conventional laser scalpel.

Pulley blocks 8 are mounted on the underside of each end portion of the arm 3, and a wire 9 is passed through the blocks 8 and is installed parallel to the optical fibre 5 so as to be slidable in the axial direction. One end of the wire 9 is fixed to the rear end of the handpiece 7, and the other end of the wire enters the unit 1 and is connected to a weight 10, which acts as a counter-balancing means for supporting the combined weight of the suspended portion of the optical fibre 5 and the handpiece 7.

Accordingly, the weight acting on the suspended portion of the optical fibre 5 is supported by the wire 9 and is counter-balanced by the weight 10, so that the handpiece 7 can be held at the desired position by movement of the wire 9 through the blocks 8. The handpiece 7 can be positioned adjacent the arm 3 when the laser scalpel is not being used. Breakage of the optical fibre 5 due to the weight of the handpiece 7 and the hanging portion of the fibre is prevented. Furthermore, the handpiece 7 and the fibre 5 do not get in the way when the unit is not being used.

Instead of the weight 10, a drum 11 as shown in Fig. 3 may be used as the balancing means. The end of the wire 9 is attached to the drum. One end of a spiral spring 12 is mounted on the inside of the drum 11, whilst the other end of the spring is fixed at the rotation axis 13 of the drum. Spring force provided by the spiral spring 12 acts to wind the wire 9 on the drum. The drum 11 is provided with a ratchet wheel 14 for stopping rotation of the drum at optional positions. The ratchet wheel 14 is stopped by means of a pawl 15. A control lever 16 of the pawl 15 can be operated from outside the unit 1.

Accordingly, when the handpiece 7 is moved downwards, the wire 9 is drawn off the drum 11, and spring force builds up in the spiral spring 12. Return rotation of the drum is prevented by the pawl 15. When the control lever 16 is operated, the ratchet is released, and the wire 9 is wound around the drum 11 by the action of the spiral spring 12, so that the handpiece 7 is lifted.

According to the present invention, therefore, an optical fibre, which forms an optical wave guide for a laser scalpel, is provided with a handpiece at its forward end which facilitates orientation of the forward end of the fibre towards the areas to be irradiated. Hence, the laser beam can be accurately directed to the target areas to be irradiated in medical treatment. Furthermore, the optical fibre is not subjected to excessive force which might break the fibre, because the handpiece and the hanging portion of the optical fibre are supported by the wire provided with balancing means. A laser scalpel of the present invention can easily be moved out of the way when other medical treatment and operations are being performed, because the handpiece and the hanging portion of the fibre can be lifted and remain in the raised position.

## Claims

1. Laser scalpel apparatus, in which an optical fibre (5) is used as an optical wave guide and the optical fibre is guided, from a unit (1) housing a laser source to areas to be irradiated, along support means (4) provided on the unit, characterised in that the optical fibre is provided with a handpiece (7) on the exterior of its free end portion, the handpiece being constructed to facilitate the directing of the free end of the fibre toward the areas to be irradiated; and supporting wire means (9) is provided along the support means so as to be axially movable, one end of the wire means being connected to the handpiece and the other end of the wire means being connected to means (10; 11—16) for counterbalancing at least the weight of the handpiece.

2. Laser scalpel apparatus as claimed in Claim 1, characterised in that the counter-balancing means comprises a counter-balance weight (10).

3. Laser scalpel apparatus as claimed in Claim 1, characterised in that the counter-balancing means comprises a spring-loaded drum for holding the wire means (9).

4. Laser scalpel apparatus as claimed in any preceding claim, characterised in that the support means (4) comprises an upright post (2) attached to the unit (1) and a horizontal arm (3) fixed to the post at the upper end thereof, the free end of the fibre (5) being suspended from the other end of the arm (3).

5. Laser scalpel apparatus as claimed in Claim 4, characterised in that the counter-balancing means (10; 11—16) counter-balances the combined weight of the handpiece (7) and the suspended portion of the fibre (5).

6. Laser scalpel apparatus as claimed in any preceding claim, characterised in that the handpiece (7) is L-shaped.

## Patentansprüche

1. Laserskalpell, bei welchem eine optische Faser (5) als optischer Wellenleiter verwendet wird und die optische Faser von einer Einheit (1), welche eine Laserquelle beinhaltet, zu der zu behandelnden Fläche mittels einer Stützeinrichtung (4) an der Einheit geführt wird, dadurch gekennzeichnet, daß die optische Faser an Ihrem äußersten freien Ende ein Handstück (7) aufweist, wobei das Handstück derart geformt ist, daß das Ausrichten des freien Endes der Faser auf die zu behandelnde Fläche erleichtert wird; und daß eine Trägerdrahteinrichtung (9) entlang der Stützeinrichtung derart vorgesehen ist, daß sie axial beweglich ist, wobei ein Ende der Drahteinrichtung mit dem Handstück und das andere Ende der Drahteinrichtung mit Einrichtungen (10; 11—16) verbunden ist, um wenigstens das Gewicht des Handstückes auszubalanzieren.

2. Laserskalpell nach Anspruch 1, dadurch gekennzeichnet, daß die Einrichtung zum Ausbalanzieren ein Gegengewicht (10) aufweist.

3. Laserskalpell nach Anspruch 1, dadurch gekennzeichnet, daß die Einrichtung zum Ausbalanzieren eine federbelastete Trommel zur Aufnahme der Drahteinrichtung (9) aufweist.

4. Laserskalpell nach einem der vorhergehenden Ansprüche, dadurch gekennzeichnet, daß die Stützeinrichtung (4) einen aufrecht stehenden Masten (2) aufweist, der an der Einheit (1) angeordnet ist und weiterhin einen horizontalen Arm (3) aufweist, der am oberen Ende des Mastes befestigt ist, wobei das freie Ende der Faser (5) von dem anderen Ende des Armes (3) herabhängt.

5. Laserskalpell nach Anspruch 4, dadurch gekennzeichnet, daß die Einrichtung zum Ausbalanzieren (10; 11—16) das Gesamtgewicht des Handstückes (7) und des herabhängenden Teiles der Faser (5) ausgleicht.

6. Laserskalpell nach einem der vorhergehenden Ansprüche, dadurch gekennzeichnet, daß das Handstück (7) L-förmig ist.

## Revendications

1. Dispositif de scalpel à laser utilisant une fibre optique (5) qui sert de guide d'ondes optique et est guidée à partir d'une unité (1) contenant une source laser vers des zones à irradier, le long d'un support (4) prévu sur l'unité, caractérisé en ce que la fibre optique est munie d'une pièce de maintien (7) disposée sur l'extérieur de la portion extrême libre de la fibre et servant à la tenir à la main, la pièce de maintien étant construite pour faciliter l'orientation de l'extrémité libre de la fibre vers les zones à irradier, et qu'un fil de support (9) est disposé axialement mobile le long du support (4, une extrémité du fil étant reliée à la pièce de maintien et son autre extrémité étant reliée à un dispositif (10; 11—16) d'équilibrage pour compenser au moins le poids de la pièce de maintien.

2. Dispositif de scalpel à laser selon la revendication 1, caractérisé en ce que le dispositif d'équi-

librage comprend un contre-poids (10).

3. Dispositif de scalpel à laser selon la revendication 1, caractérisé en ce que le dispositif d'équilibrage comprend un tambour chargé par ressort pour tenir le fil (9).

4. Dispositif de scalpel à laser selon l'une quelconque des revendications précédentes, caractérisé en ce que le support (4) comporte un montant vertical (2) attaché à l'unité (1) et un bras horizontal (3) fixé au sommet du montant, l'extrémité libre de la fibre (5) étant suspendue de l'autre extrémité du bras (3).

5. Dispositif de scalpel à laser selon la revendication 4, caractérisé en ce que le dispositif d'équilibrage (10; 11—16) compense le poids combiné de la pièce de maintien (7) et de la portion suspendue de la fibre (5).

6. Dispositif de scalpel à laser selon l'une quelconque des revendications précédentes, caractérisé en ce que la pièce de maintien (7) est en forme de L.

*Fig. 1*

*Fig. 2*

*Fig. 3*